# EUROPEAN PATENT SPECIFICATION

(11) **EP 2 197 459 B1**
(45) Date of publication and mention of the grant of the patent: **06.01.2016**
(21) Application number: 08794131.6
(22) Date of filing: 15.09.2008
(51) Int. Cl.: A61K 35/50, C07K 14/785

(54) **A LUNG-SURFACTANT PRODUCT**
LUNGEN-TENSIDPRODUKT
PRODUIT SURFACTANT PULMONAIRE

(30) Priority: 14.09.2007 SE 0702049
(43) Date of publication of application: 23.06.2010
(73) Proprietor: Larsson, Marcus, 237 34 Bjärred (SE); Herbst, Andreas, 237 34 Bjärred (SE)
(72) Inventor: Larsson, Marcus, 237 34 Bjärred (SE); Herbst, Andreas, 237 34 Bjärred (SE)
(74) Representative: Brann AB
(86) International application number: PCT/SE2008/000511
(87) International publication number: WO 2009/035395

(56) References cited:
- EP-B1- 0 491 033
- WO-A1-87/02037
- US-A- 5 227 308
- HALLMAN M. ET AL.: 'Isolation of human surfactant from amniotic fluid and a pilot study of its efficacy in respiratory distess syndrome' PEDIATRICS vol. 71, no. 4, 1983, pages 473 - 482, XP003024234
- LARSSON M. ET AL.: 'Enhanced efficacy of porcine lung surfactant extracted by utilization of its aqueous swelling dynamics' CLIN. PHYSIOL. & FUNC. IM. vol. 22, 2002, pages 39 - 48, XP003024235
- DUCK-CHONG C.G. ET AL.: 'Sedimentation of lung-derived phospholipid during low-speed centrifugration of amniotic fluid' CLINICAL CHEMISTRY vol. 27, no. 8, 1981, pages 1424 - 1426, XP003024236
- RUIZ-BUDRIA J. ET AL.: 'Effect of centrifugation on amniotic fluid phospholipid composition' REVISTA ESPANOLA DE FISIOLOGIA vol. 48, no. 3, 1992, pages 203 - 210, XP003024237

## Description

### FIELD OF INVENTION

The invention relates to an isolated lung-surfactant product comprising lung-surfactant and an amniotic aqueous solvent being substantially cell debris and vernix free, a method for the production of said lung-surfactant product.

### BACKGROUND OF INVENTION

Pulmonary surfactant is a complex mixture of lipids and proteins synthesized and secreted by type II pneumocytes in the alveolar lining. The role of this surfactant is to decrease the work associated with breathing by reducing the surface tension at the air-water interface in the lung. The main constituents of pulmonary surfactant are lipids and proteins, which accounts for about 90 % and 10% of the surfactant dry weight, respectively. Of the lipids, approximately 85-90% are phospholipids of which 80% are phosphatidylcholine (PC) and about 10 % are phosphatidylglycerol lipids (PG). Cholesterol dominates among the other lipids [1].

The surfactant proteins SP-A, SP-B, SP-C, and SP-D can be divided into two sub-classes. SP-A and SP-D are large hydrophilic oligomeric proteins, structurally related to the proteins of the complement system. They have a role in the innate host defence but also have profound effects on lung surfactant ultra-structure and physiological function in-vivo. SP-B and SP-C are small hydrophobic proteins and are mainly responsible for maintaining the ultra-low surface tension required in the lung for normal breathing. The surfactant proteins have proven to be very difficult to produce synthetically [2].

A number of diseases are caused by a disturbed amount of the pulmonary surfactant or even complete absence of the pulmonary surfactant, such as in Infant Respiratory Distress Syndrome, IRDS. Today these infants can be treated by intra-tracheal instillation of lung surfactant extracts from porcine of bovine origin.

Some previous works explored the possibilities of using human lung surfactant purified from amniotic fluid, where even clinical trials were performed [3]. This latter was, however, discontinued in the early 1990's. The protocols for surfactant extraction employed by Hallman et.al involved several steps of dilution and re-suspension of the surfactant fraction in various non-physiological solutions. Most important, however, the solutions were neither isotonic, nor had the necessary di-valent ions present to maintain the crucial interactions between the surfactant lipids, SP-B, SP-C and SP-A. The effect of SP-A is dependant on the presence of calcium ions. Moreover, the purification procedure used in these earlier reports did not address the structural effects induced by air on the lung surfactant system.

US 5 227 308 discloses a method for assessing lung maturity in a fetus/infant, whereby the sample of amniotic fluid or lung lavage, after removal of particles, is mixed with the fluorescent indicator Laurdan. However, the centrifugation step in example 2 does not separate vernix into the pellet and the amniotic fluid supernatant material obtained after the first centrifugation step make it unusable as a lung surfactant product since the amount of the surfactant per volume is insufficient for therapeutical use.

WO 87/02037 relates to proteins for enhancing pulmonary surfactant activity and to their use in the treatment of infants afflicted with RDS. The disclosure shows the isolation of proteins from alveolar proteinosis, where the clearance of lung surfactants from the lung is disturbed. Due to lung lavage, the retrieved material is diluted and suspended in a solution lacking divalent ions , such as calcium as well as has been subjected to air which makes is not suitable as a lung surfactant product for therapeutical use.

EP 0 491 033 relates to lung surfactant proteins and to the process of isolating and purifying recombinant lung surfactant from individuals suffering from RDS. Again such a product is not suitable to be used for therapeutical use.

Our invention is based on the approach that at all times the surfactant fraction of interest is handled in the original physiological vehicle, i.e., the amniotic fluid, whereby a number of components of importance will be preserved during the procedure. Moreover, by avoiding processing steps involving the addition of any other medium than the original physiological vehicle, i.e. the amniotic fluid, and by avoiding the exposure to large air interfaces, structural changes on the ultra-structural level of the lung surfactant fraction can be avoided. Therefore, a novel process was developed using processing steps in which no other components were added to the amniotic fluid and at all times minimizing the exposure to large air interfaces, which resulted in a novel amniotic fluid fraction having superior properties, such as having a markedly higher surface activity compared to lung surfactant extracts available on the market today.

### SUMMARY OF THE INVENTION

The object of the present invention is to provide a new improved lung-surfactant product having unique properties which for the first time enables the possibility to treat a patient in need thereof with such a product, able to rapidly establish a low-energy surface phase towards air. The isolated lung-surfactant product, being manufactured by a process which minimizes air-exposure, in order to keep the extract in its physiological ideal state. The isolated lung-surfactant product being a pharmaceutical composition for the treatment of a number of disorders or diseases in which the patient suffers from a lung disease.

The invention relates in one aspect to an isolated lung-surfactant product comprising lung-surfactants and an amniotic aqueous solvent being substantially cell debris and vernix free.

In a second aspect, the invention relates to an isolated lung-surfactant product obtainable by the process comprising the steps of; providing amniotic fluid from at least one mammal, removing cells, cell debris, and vernix from said amniotic fluid, ultracentrifugating said amniotic fluid and obtaining a pellet and a supernatant, discarding about 70-99 % of the supernatant such as 75, 80, 85 or at least 90% of said supernatant comprising said amniotic fluid and obtaining a lung-surfactant product comprising lung-surfactants and an amniotic aqueous solvent being substantially cell debris and vernix free.

In a third aspect the invention relates to a method of producing a lung-surfactant product comprising the steps of providing amniotic fluid from at least one mammal, removing cells and cell debris from said amniotic fluid, ultracentrifugating said amniotic fluid and obtaining a pellet and a supernatant, discarding at least 90% of said supernatant comprising said amniotic fluid and obtaining a lung-surfactant product comprising lung-surfactants and an amniotic aqueous solvent being substantially cell debris and vernix free.

By providing said isolated lung-surfactant product, the possibility for a number of patients to be successfully treated for their lung disorder or disease is increased and thereby achieving an increased health status of said patients.

Further advantages and objects with the present invention will be described in more detail, inter alia with reference to the accompanying figures.

### BRIEF DESCRIPTION OF THE DRAWINGS

Fig 1. Cryo-TEM image of one centrally situated lung surfactant vesicle forming connections with the surrounding lung surfactant vesicles, where the interactions between these vesicles distort the usual spherical shapes into the formation of apparently planar interfaces between adjacent vesicles.
Fig. 2. Cryo-TEM, lower magnification providing overview of how the interactions between adjacent vesicles distort these into shapes with circular-cylindrical appearances with parallel internal walls of these distorted LB's.
Fig. 3. TEM image showing the apparently planar connections between apposing LB's with distinct stacking of the lipid bilayers.
Fig. 4. Polarization microscopy image showing the typically observed surface enlargement of the air/water interface. The surface structure is highly birefringent, indicating liquid crystalline order, and the establishment of a coherent surface phase at the air/water interface.

### DETAILED DESCRIPTION OF THE INVENTION

### Definitions

In the context of the present application and invention, the following definitions apply:
The term "lung surfactant" is intended to mean the lipid-protein complex secreted by the alveolar type II cells.
The term "low-energy surface phase towards air" is intended to mean a surface structure with low net-surface tension formed by lung surfactant with a liquid crystalline order in the terminal air-spaces of mammalian lungs.
The term "aggregates/vesicles" is intended to mean the closed membrane structures formed by lung surfactant in an aqueous medium.
The term "elongated" is intended to mean the relationship between the longest appearing planar aspect of a closed membrane assembly in relation to the radius of the semi-circular end regions with the longest appearing planar aspect being more than 4 times the average end-radius. The form of these closed membranes can also be regarded as perfect circular-cylindrical shapes.
The "term lamellar bodies (LB's)" is intended to mean the multilamellar vesicles, containing lung surfactant secreted by alveolar type II cells.

### The isolated lung-surfactant product

The invention relates to an isolated lung-surfactant product comprising lung-surfactants and an amniotic aqueous solvent being substantially cell debris and vernix free. The term "substantially" is intended to mean that all visible cell debris as well as vernix are removed. The amniotic aqueous solvent being used as the sole solvent during the process in which the lung-surfactants are produced/isolated from amniotic fluid from one or more mammals, such as human beings. The amniotic fluid keeps the lung-surfactants in their natural environment as well as protects them from air. By keeping the lung-surfactants preparations in such an environment, their natural properties are kept, which upon use on a patient will result in a significant clinical effect.

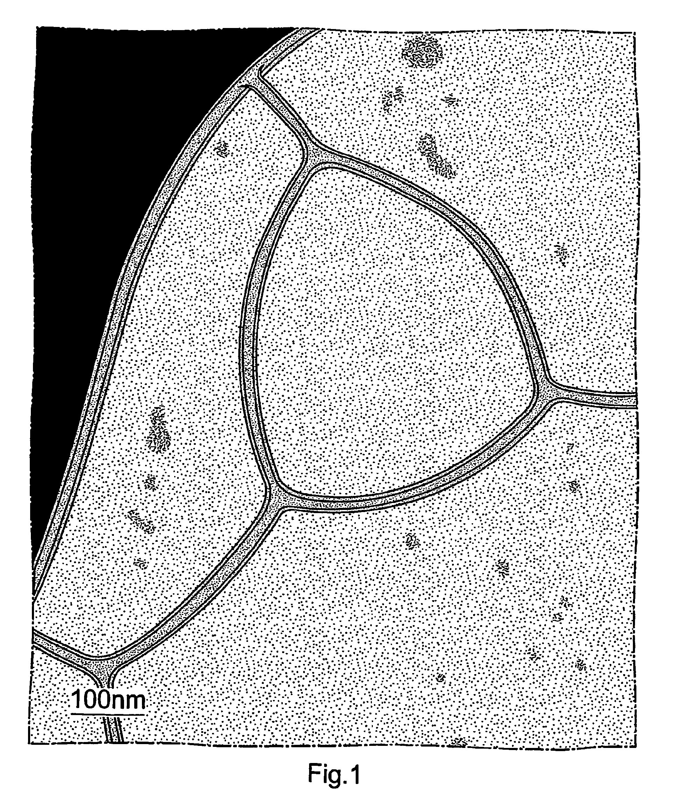

The isolated lung-surfactants in said product forms uniquely shaped contact interfaces between adjacent lung surfactant vesicles/aggregates (c.f. figures 1, 2 and 3), which are seen as the occurrence of circular-cylindrical elongated structures which exhibit continuous contact interfaces between adjacent circular cylindrical elongated structures.

Said lung surfactant comprises phospholipids and lung surfactant associated proteins. The amniotic fluid fraction may be obtained from any mammal, such as human, pig or cow, etc. The mammal may be genetically modified. One example being that the amniotic fluid fraction is obtained from a human being. The amniotic fluid is collected during caesarean section by a catheter or syringe when the foetal membranes are opened. When such amniotic fluid from one full term pregnancy is processed by our novel protocols, the yield is rather high, and the obtainable amount of lung-surfactant product from one mother/child donor is more than sufficient for one typical treatment dose of lung surfactant. Thus, by using our protocols, surfactant needs not to be pooled from several donors, thereby reducing the risk of infectious disease transmission. On a side note, in most developed countries, all presumptive donors of amniotic fluid (expecting mothers) are routinely tested for the common blood borne infections (i.e. HIV, Hepatitis B and C). However, the possibility of blood borne disease transmission is still a risk-factor, and this factor must be minimized.

By keeping the lung-surfactants in its physiological vehicle/environment at all times during the purification procedure, the lung-surfactants can be considered to be in statu nascendi. Thus, it is in the same form as is present in the newborn child at the time of the first breath. This novel lung-surfactant fraction has been shown to exhibit superior surface-active properties, described below.

The lung-surfactant product comprises a number of proteins including SP-B and SP-C in the lipid bilayers and at least surfactant protein A (SP-A) in the aqueous phase. SP-A is a large, water-soluble glycoprotein with a collage-like N-terminus and variable glycosylation of the C-terminus. Trimers comprising 35 kDa monomers associate into octadecamers with a molecular mass of approximately 700 kDa. SP-A has an isoelectric point of 4-5 and is thus negatively charged at physiological pH. The amniotic fluid extract may also comprise surfactant protein D (SP-D). SP-D being a member of the Ca²⁺- dependent carbohydrate binding collectin family. SP-D is a dodecameric protein and being involved in the innate defence system. Both SP-A and SP-D are hydrophilic. SP-B and SP-C are small hydrophobic proteins that both are crucial for the physiological activity of lung surfactant. Furthermore, SP-B and SP-C are able to link apposing lipid bilayers. Moreover, the invented lung-surfactant product contains Ca²⁺ as well as all the components that are found in the amniotic fluid except for the cells, cell debris as well as the vernix which are substantially reduced or removed from the lung-surfactant product.

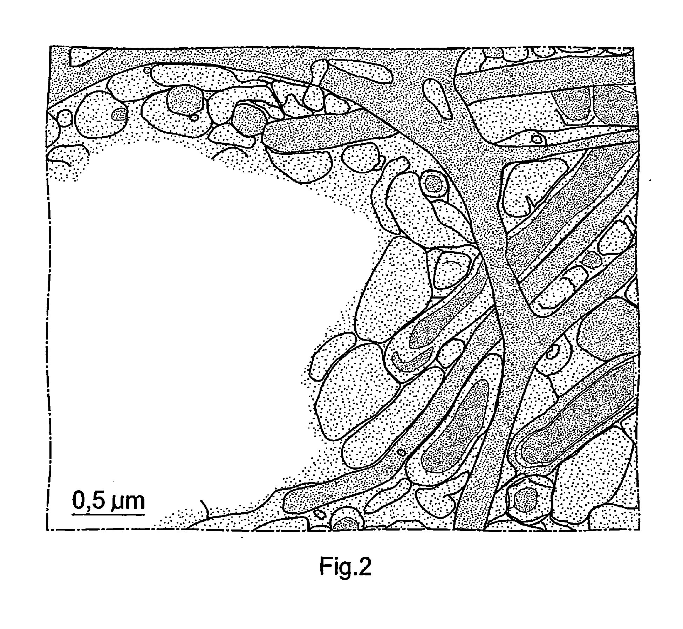

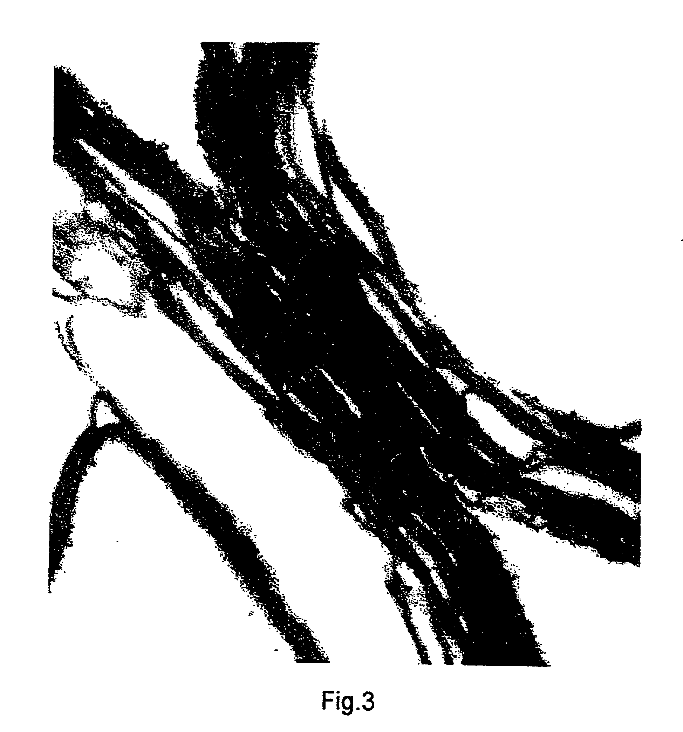

The amniotic fluid fraction comprises lipids and proteins that form aggregates/vesicles. The extract has unique surface-active properties, ideal for rapidly establishing a surface-phase, providing a low-energy surface towards air. Characteristics of the extract on the ultra-structural scale is seen in the cryo-transmission electron microscope (see figure 1 and 2) as a distinct tendency of vesicles and multi-lamellar vesicles (Lamellar bodies, LB's) to adhere to one another, thereby creating rather planar interfaces, and also producing quite elongated vesicles with apparently planar borders (as seen in the electron microscope) with a length scale of about or more than four times the end radius of the curvature at the end of such an elongated vesicle/LB. Furthermore, the membranes contained in some LB's can be seen to strongly interact, creating stacked membranes (figures 2 and 3) following the planar orientation of the outermost layer of the LB.

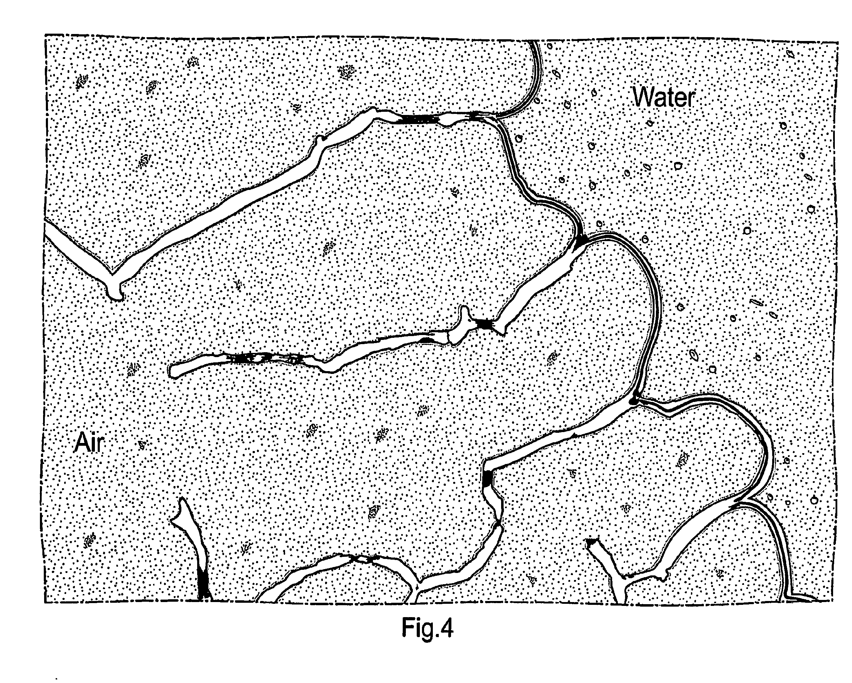

When observing the extract in the polarizing microscope (figure 4), a remarkable phenomenon of surface area enlargement at the air/water (amniotic fluid surfactant phase) interface is seen. In this process, this interface exhibits an increasing birefringence over time, indicating the establishment of a liquid crystalline order of macroscopic dimensions (a surface phase) at the interface towards air. Furthermore, LB's can be seen in the light microscope to transform into this surface phase in the timescale of seconds to minutes. The fluid border is pushed back and a highly birefringent, tree-like network is formed. This phenomenon is observed within 0-5 minutes after the sample is put on a microscopy slide with a coverslip on. This behaviour is a constant characteristic of the product. Such surface area enlargement phenomena in a lung surfactant has previously been shown to correlate to higher therapeutic efficacy (resulting in higher arterial blood oxygen levels) of a porcine lung surfactant studied in a rat lung injury model [4]. In this latter case, however, the phenomenon of surface area increase was only present in a rather short timespan (minutes) after sample preparation.

The invented lung-surfactant product may be a pharmaceutical composition which solely contains the lung-surfactant product or a pharmaceutical composition which also contains other components such as antibiotics, growth factors etc. Examples of antibiotic agents are found below. The invented lung surfactant product may be used to increase the efficacy of presently available lung surfactant extracts (natural lung surfactants, semisynthetic lung surfactants and synthetic lung surfactants) by doping them with the lung surfactant of the present invention.

The invention also relates to an isolated lung-surfactant product obtainable by the process comprising the steps of; providing amniotic fluid from at least one mammal, removing cells and cell debris from said amniotic fluid, ultracentrifugating said amniotic fluid and obtaining a pellet and a supernatant, , discarding about 70-99 % of the supernatant such as 75, 80, 85 or at least 90% of said supernatant comprising said amniotic fluid and obtaining a lung-surfactant product comprising lung-surfactants and an amniotic aqueous solvent being substantially cell debris and vernix free. The method will be described in details below.

### A process to obtain said isolated lung-surfactant product

The invention also relates to a new improved process to enable the possibility to obtain the new invented lung-surfactant product.

The method uses amniotic fluid as source material. The amniotic fluid may be obtained from at least one mammal, such as a human being, a pig or a cow. The amniotic fluid may be collected at planned caesarean section before membrane rupture, preferentially at or near term gestation when pulmonary maturation is completed and the amniotic fluid is containing high amounts of surfactant. The amniotic fluid may be obtained by the use of a syringe with a needle or a plastic catheter inserted through the foetal membranes during caesarean section at sterile conditions immediately before delivering the fetus. The amniotic fluid may then be stored and transported at 4 °C. The amniotic fluid is preferably free from macroscopic blood and meconium contamination. Furthermore, the amniotic fluid is preferably handled with minimum exposure to large air-surfaces prior to the fractionation/purification. Also, syringes, catheters and collecting tubes is preferably as inert as possible, specifically avoiding use of such materials which have lubricated rubber surfaces, which may result in surfactant inactivation.

The invented method of producing a lung-surfactant product comprising the steps of; providing amniotic fluid from at least one mammal, removing cells and cell debris from said amniotic fluid, ultracentrifugating said amniotic fluid and obtaining a pellet and a supernatant, , discarding about 70-99 % of the supernatant such as 75, 80, 85 or at least 90% of said supernatant comprising said amniotic fluid and obtaining a lung-surfactant product comprising lung-surfactants and an amniotic aqueous solvent being substantially cell debris and vernix free. As an example a sigma 3-18K may be used to remove the cells and cell debris. The ultracentrifugation may be done at from about 10 000 x g to about 50 000 x g, from about 10 000 x g to about 25 000 x g, from about 12 000 x g to about 17 000 x g for about 5 to about 15 minutes or at 15 000 x g for 10 minutes. The step to remove cells and cell debris may be done at about 400 to about 800 x g or alternatively at 500 x g for 5 minutes. If necessary an additional ultracentrifugation step may be used. The additional ultracentrifugation step may be performed at about 15 000 to about 36 000 x g or at 20 000 x g for 1 minute.

The temperature to be used during the processing of the amniotic fluid may vary and be from about 2 to about 12 degrees C, such as at 10 degrees C.

The supernatant will always be removed carefully and a thin layer of amniotic fluid left on top of the whitish pellet (to protect from drying and air-exposure. By such a process, a pellet is obtained having the characteristics of the amniotic fluid fraction defined above. The thin layer of amniotic fluid which is left on top of the pellet, and the water phase contained in the structure, keeps the amniotic fluid extract in its originally physiological environment. The process can be performed rather free from air to keep the amniotic fluid in its physiological environment as much as possible. Further processing steps may also be performed to alter the viscosity, e.g. re-suspension with amniotic fluid, treatment with DNAse, a washing step using a physiologically balanced ion containing solution such as Ringer's solution, to name a few such steps. Also, addition of antibiotics, such as penicillin or gentamicin for conservation and therapeutic purposes is possible. Gamma ray irradiation of the extract can be performed to sterilize the product. However, the entire process should preferably be sterile. Sterilization of the lung-surfactant product must take into account the preservation of the internal membrane structures and related proteins.

### REFERENCES

1. J. Pérez-Gil, Molecular Interactions in Pulmonary Surfactant Films, Biol Neonate 2002;81(suppl 1):6-15.
2. T. Curstedt, J Johansson, New Synthetic Surfactants - Basic Science, Biol Neonate 2005;87:332-337.
3. M. Hallman et al., Isolation of Human Surfactant from Amniotic Fluid and a Pilot Study of its Efficacy in Respiratory Distress Syndrome, Pediatrics 1983;71(No.4):473-482.
4. Larsson M, Haitsma JJ, Lachmann, B, Larsson K, Nylander T, Wollmer P. Enhanced efficacy of porcine lung surfactant extract by utilization of its aqueous swelling dynamics. Clin Physiol & Func Im. 2002; 22: 39 - 48.

### EXAMPLES

### EXAMPLE 1

### Isolation of amniotic fluid fraction

Amniotic fluid was collected from healthy pregnant women at term caesarean delivery for uncomplicated causes (breech presentation, previous caesarean delivery, or fear of giving birth), after informed consent. Directly after incision of the uterine wall, a sterile silicon catheter is inserted through the fetal membranes, and amniotic fluid is aspirated immediately before delivering the baby. Sterile 20 cc non-heparinised syringe, free from surface active lubricants (B. Braun, Meslungen, Germany) to collect the amniotic fluid. An alternative method which also was used was to puncture the fetal membranes with a sterile cannula immediately after the incision of the uterine wall, using the same type of syringe for aspiration. In order to achieve larger volumes of amniotic fluid, a closed system with a sterile cannula connected to a syringe and a collecting plastic bag may be used. About 25-50ml amniotic fluid was collected.

The amniotic fluid samples were stored in sterile tubes, in refrigerator at 4°C until processing.

The amniotic fluid fraction was produced by the following steps:
1. Centrifugation step for removal of cells and cellular debris at 500 g for 5 minutes at 10°C using a Sigma 3-18K.
2. Retrieval of the cell and cell debris free amniotic fluid supernatant. Also removal of free floating sebum fat aggregates (originating from the foetal skin) by pipetting using a plastic pipette such as a Finnpipette 250 microliter universal tip.
3. Ultracentrifugation step at 15000 g at 10 degrees Celsius for ten minutes of supernatant from step 2 using a Sigma 3-18K.
4. Supernatant from step 3 was removed.
5. Surfactant pellet produced by step 3 was collected (with this pellet, small amounts of amniotic fluid are also included, keeping the pellet from drying).
6. This viscous pellet material collected in step 5 was subjected to yet another centrifugation step at 19000 g for one minute at 10 degrees Celsius in a Sigma microfuge.
7. The surfactant surface phase pellet is then stored in an Eppendorf 1,5ml tube with a small amount of excess amniotic fluid to protect from drying.
8. Should the surfactant pellet in step 7 appear too viscous, a washing step may be included, wherein said washing is performed by the use of Ringer's solution.

The surfactant end product will contain some Surfactant Protein A (can be identified with e.g. anti-human surfactant protein A, polyclonal antibody, Chemicon International, catalogue number AB3420). The surfactant end product was stored at 4°C.

### EXAMPLE 2

### Cryo-Transmission Electron Microscopy, Cryo-TEM

Cryo transmission electron microscopy (cryo-TEM) is a rather artifact-free method, well suited to study the alveolar surfactant system. The sample of lung surfactant material prepared according to example 1, was spread on the cryo-grid in minute amounts (5µl) using a pipette and thereafter vitrified by immediately being plunged into liquid ethane (-180°C). The samples were vitrified at 37°C and 100% humidity. The samples were viewed in a Philips Bio-twin 120 cryo with a La₂B₆ filament. The cryo-TEM samples were at all times kept below -180°C. Images were recorded using a Gatan CCD with Digital Micrograph 3.31 software for Macintosh.

### EXAMPLE 3

### Polarizing microscopy

An Olympus CX-41 microscope equipped with a polarizer and 4X, 10X and 40X objectives was used. Images were captured using a Nikon DS2Mv CCD camera and Nikon NIS software for Microsoft Windows. The sample holder was kept at 37°C. About 5 microliters of lung surfactant phase prepared according to example 1, was put on the microscopy slide and the coverslip put on and thereafter the air/water interface was continually observed in polarization mode.

## Claims

1. An isolated lung-surfactant product comprising
a. lung-surfactants and
b. an amniotic aqueous solvent being substantially cell debris and vernix free.

2. The isolated lung-surfactant product according to claim 1, wherein a significant part of said lung-surfactant phase in its aqueous amniotic solvent forms closed circular cylindrical elongated structures.

3. The isolated lung-surfactant product according to claim 2, wherein said closed aggregates are laterally associated thereby forming interfaces appearing planar.

4. An isolated lung-surfactant product obtainable by a process which eliminates or reduces the contact with air comprising the steps of:
a. Providing isolated amniotic fluid discarded during a caesarean section,
b. Removing cells and cell debris from said amniotic fluid,
c. Removing vernix from said amniotic fluid,
d. Ultracentrifugating said amniotic fluid and obtaining a pellet and a supernatant,
e. Discarding , about 70-99 % of the supernatant such as at least 90% of said supernatant comprising said amniotic fluid and
f. Obtaining a lung-surfactant product comprising lung-surfactants and an amniotic aqueous solvent being substantially cell debris and vernix free.

5. A pharmaceutical composition comprising said lung-surfactant product according to any of preceding claims.

6. A method of producing a lung-surfactant product which minimizes the contact with air comprising the steps of:
a. Providing isolated amniotic fluid discarded during a caesarean section,
b. Removing cells and cell debris from said amniotic fluid,
c. Removing vernix from said amniotic fluid,
d. Ultracentrifugating said amniotic fluid and obtaining a pellet and a supernatant,
e. Discarding , about 70-99 % of the supernatant such as at least 90% of said supernatant comprising said amniotic fluid and
f. Obtaining a lung-surfactant product comprising lung-surfactants and an amniotic aqueous solvent being substantially cell debris and vernix free.
g. at all times keeping the lung surfactant product in an excess amount of the aqueous solvent to protect the lung surfactant from air-exposure.

7. The method according to claim 6, wherein step c) is done at from about 10 000 x g to about 50 000 x g for about 5 to about 15 minutes.

8. The method according to any of claims 6-7, wherein step b) is done at about 400 to about 800 x g for about 4 to about 10 minutes.

9. The method according to claim 8, wherein step b) is done at 500 x g for 5 minutes.

10. The method according to any of claims 6-9, wherein an additional ultracentrifugating step f) is included.

11. The method according to claim 10, wherein step f) is done at about 15 000 to about 36 000 x g from about 30 second to 5 minutes.

12. The method according to any of claims 6-11, wherein the steps are performed at a temperature from about 2 to about 12 °C.

13. The method according to any of claims 6-12, wherein a washing step using a physiologically balanced ion containing washing solution is performed.

## Patentansprüche

1. Isoliertes Lungensurfactant-Produkt, umfassend
a. Lungensurfactants und
b. ein amniotisches wässriges Lösungsmittel, das im Wesentlichen frei von Zelltrümmern und Vernix caseosa ist.

2. Isoliertes Lungensurfactant-Produkt nach Anspruch 1, wobei ein signifikanter Teil der Lungensurfactant-Phase in ihrem wässrigen amniotischen Lösungsmittel geschlossene, ringförmige, zylindrische, längliche Strukturen bildet.

3. Isoliertes Lungensurfactant-Produkt nach Anspruch 2, wobei die geschlossenen Aggregate lateral verbunden sind, wodurch sie Grenzflächen bilden, die sich planar manifestieren.

4. Isoliertes Lungensurfactant-Produkt, das durch ein Verfahren erhalten werden kann, das den Kontakt mit Luft eliminiert oder reduziert, umfassend die Schritte:
a. Bereitstellen von isoliertem Fruchtwasser, das während eines Kaiserschnitts verworfen wurde,
b. Entfernen von Zellen und Zelltrümmern aus dem Fruchtwasser,
c. Entfernen von Vernix caseosa aus dem Fruchtwasser,
d. Ultrazentrifugieren des Fruchtwassers und Erhalten eines Pellets und eines Überstands,
e. Verwerfen von etwa 70-99% des Überstands, beispielsweise mindestens 90% des Überstands, der das Fruchtwasser umfasst, und
f. Erhalten eines Lungensurfactant-Produkts, das Lungensurfactants und ein amniotisches wässriges Lösungsmittel umfasst, das im Wesentlichen frei von Zelltrümmern und Vernix caseosa ist.

5. Arzneimittel, das das Lungensurfactant-Produkt nach einem der vorangehenden Ansprüche umfasst.

6. Verfahren zur Herstellung eines Lungensurfactant-Produkts, das den Kontakt mit Luft minimiert, umfassend die Schritte:
a. Bereitstellen von isoliertem Fruchtwasser, das während eines Kaiserschnitts verworfen wurde,
b. Entfernen von Zellen und Zelltrümmern aus dem Fruchtwasser,
c. Entfernen von Vernix caseosa aus dem Fruchtwasser,
d. Ultrazentrifugieren des Fruchtwassers und Erhalten eines Pellets und eines Überstands,
e. Verwerfen von etwa 70-99% des Überstands, beispielsweise mindestens 90% des Überstands, der das Fruchtwasser umfasst, und
f. Erhalten eines Lungensurfactant-Produkts, das Lungensurfactants und ein amniotisches wässriges Lösungsmittel umfasst, das im Wesentlichen frei von Zelltrümmern und Vernix caseosa ist,
g. permanentes Halten des Lungensurfactant-Produkts mit einer überschüssigen Menge des wässrigen Lösungsmittels, um das Lungensurfactant vor Luftexposition zu schützen.

7. Verfahren nach Anspruch 6, wobei Schritt c) von etwa 10 000 x g bis etwa 50 000 x g für etwa 5 bis etwa 15 Minuten durchgeführt wird.

8. Verfahren nach einem der Ansprüche 6 bis 7, wobei Schritt b) von etwa 400 bis etwa 800 x g für etwa 4 bis etwa 10 Minuten durchgeführt wird.

9. Verfahren nach Anspruch 8, wobei Schritt b) mit 500 x g für 5 Minuten durchgeführt wird.

10. Verfahren nach einem der Ansprüche 6 bis 9, wobei ein zusätzlicher Ultrazentrifugierungsschritt f) umfasst ist.

11. Verfahren nach Anspruch 10, wobei Schritt f) mit etwa 15000 bis etwa 36000 x g für etwa 30 Sekunden bis 5 Minuten durchgeführt wird.

12. Verfahren nach einem der Ansprüche 6 bis 11, wobei die Schritte bei einer Temperatur von etwa 2 bis etwa 12°C durchgeführt werden.

13. Verfahren nach einem der Ansprüche 6 bis 12, wobei ein Waschschritt unter Verwendung einer physiologisch ausgeglichenen Waschlösung, die Ionen enthält, durchgeführt wird.

## Revendications

1. Produit surfactant pulmonaire isolé comprenant
a. des surfactants pulmonaires et
b. un solvant aqueux amniotique sensiblement dépourvu de débris cellulaires et de *vernix.*

2. Produit surfactant pulmonaire isolé selon la revendication 1, dans lequel une partie importante de ladite phase de surfactant pulmonaire dans son solvant amniotique aqueux forme des structures allongées cylindriques circulaires fermées.

3. Produit surfactant pulmonaire isolé selon la revendication 2, dans lequel lesdits agrégats fermés sont associés latéralement, formant de ce fait des interfaces d'apparence plane.

4. Produit surfactant pulmonaire isolé pouvant être obtenu par un procédé qui élimine ou réduit le contact avec l'air comprenant les étapes de:
a. fourniture de liquide amniotique isolé éliminé au cours d'une césarienne,
b. retrait de cellules et de débris cellulaires dudit liquide amniotique,
c. retrait du *vernix* dudit liquide amniotique,
d. ultracentrifugation dudit liquide amniotique et obtention d'un culot et d'un surnageant,
e. élimination d'environ 70 à 99 % du surnageant tel qu'au moins 90 % dudit surnageant comprenant ledit liquide amniotique et
f. obtention d'un produit surfactant pulmonaire comprenant des surfactants pulmonaires et un solvant amniotique aqueux sensiblement dépourvu de débris cellulaires et de *vernix.*

5. Composition pharmaceutique comprenant ledit produit surfactant pulmonaire selon l'une quelconque des revendications précédentes.

6. Procédé de production d'un produit surfactant pulmonaire qui minimise le contact avec l'air, comprenant les étapes de :
a. fourniture de liquide amniotique isolé éliminé au cours d'une césarienne,
b. retrait de cellules et de débris cellulaires dudit liquide amniotique,
c. retrait du vernix dudit liquide amniotique,
d. ultracentrifugation dudit liquide amniotique et obtention d'un culot et d'un surnageant,
e. élimination d'environ 70 à 99 % du surnageant tel qu'au moins 90 % dudit surnageant comprenant ledit liquide amniotique et
f. obtention d'un produit surfactant pulmonaire comprenant des surfactants pulmonaires et un solvant amniotique aqueux sensiblement dépourvu de débris cellulaires et de *vernix,*
g. maintien en permanence du produit surfactant pulmonaire dans une quantité en excès de solvant aqueux pour protéger le surfactant pulmonaire d'une exposition à l'air.

7. Procédé selon la revendication 6, dans lequel l'étape c) est effectuée à environ 10 000 x g à environ 50 000 x g pendant environ 5 à environ 15 minutes.

8. Procédé selon l'une quelconque des revendications 6 à 7, dans lequel l'étape b) est effectuée à environ 400 à environ 800 x g pendant environ 4 à 10 minutes.

9. Procédé selon la revendication 8, dans lequel l'étape b) est effectuée à 500 x g pendant 5 minutes.

10. Procédé selon l'une quelconque des revendications 6 à 9, dans lequel une étape f) d'ultracentrifugation supplémentaire est incluse.

11. Procédé selon la revendication 10, dans lequel l'étape f) est effectuée à environ 15 000 à environ 36 000 x g pendant environ 30 secondes à environ 5 minutes.

12. Procédé selon l'une quelconque des revendications 6 à 11, dans lequel les étapes sont réalisées à une température d'environ 2 à environ 12 °C.

13. Procédé selon l'une quelconque des revendications 6 à 12, dans lequel on réalise une étape de lavage utilisant une solution de lavage contenant des ions physiologiquement équilibrée.
